(19) Europäisches Patentamt
European Patent Office
Office européen des brevets

(11) Publication number: **0 227 100 B1**

(12) # EUROPEAN PATENT SPECIFICATION

(45) Date of publication of patent specification: **03.04.91**

(51) Int. Cl.5: **C07D 233/60**, C07D 233/61, C07D 249/08, C07D 233/90, C07D 233/94, C07D 233/64, C07D 401/06, C07D 403/12, C07D 405/12, C07D 405/14, A61K 31/415

(21) Application number: **86117943.0**

(22) Date of filing: **23.12.86**

(54) **N-Vinylimidazoles and -triazoles and pharmaceutical compositions containing them.**

(30) Priority: **25.12.85 JP 294905/85**

(43) Date of publication of application:
**01.07.87 Bulletin 87/27**

(45) Publication of the grant of the patent:
**03.04.91 Bulletin 91/14**

(84) Designated Contracting States:
**CH DE FR GB IT LI SE**

(56) References cited:
**EP-A- 0 076 628**
**EP-A- 0 142 190**
**EP-A- 0 217 199**
**DE-A- 3 313 499**
**GB-A- 2 054 560**
**GB-A- 2 118 549**
**EP-A- 0 079 856**
**EP-A- 0 162 359**
**EP-A- 0 227 011**
**FR-A- 2 557 874**
**GB-A- 2 088 872**
**US-A- 4 328 348**

(73) Proprietor: **SHIONOGI SEIYAKU KABUSHIKI KAISHA**
**12, Dosho-machi, 3-chome Higashi-ku,**
**Osaka-shi**
**Osaka 541(JP)**

(72) Inventor: **Ogata, Masaru**
**8-20-8, Sumiyoshiyamate Higashinada-ku**
**Kobe-shi(JP)**
Inventor: **Tawara, Katsuya**
**1-1-814, Higashiota**
**Ibaraki-shi Osaka(JP)**
Inventor: **Ueda, Motohiko**
**7-16, Minamitakahama-cho**
**Suita-shi Osaka(JP)**
Inventor: **Sato, Kosaburo**
**5-15, Koshienabiki-cho**
**Nishinomiya-shi Hyogo(JP)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 227 100 B1

## Description

The present invention relates to N-vinylimidazoles and N-vinyltriazoles which are useful cardiotonic drugs.

In US-A-4-328 348 and the corresponding GB-A-2 054 560 benzylimidazoles are described which are useful as antimycotic drugs or agricultural fungicides.

In GB-A-2 088 872 l-benzylimidazoles are described which are useful as antimycotic agents and/or agricultural bactericides.

In FR-A-2 557 874 l-vinylimidazoles are described which are useful as antimycotic agents.

In GB-A-2 118 549 benzylimidazoles are described which are useful as antimycotic drugs or agricultural funigicides. In EP-A-162 359 1-vinylimidazoles are described which are useful as antimycotic drugs or agricultural fungicides. In EP-A-76 628 1-vinylimidazolyl- and 1- and 4-vinyl-(1,2,4-triazolyl)-compounds are described which exhibit fungicidal activity against phytopathogenic fungi.

In EP-A-217 199 (Art. 54(3) EPC) alkenylimidazoles and -triazoles are described which are antimycotic agents and fungicides.

The present invention relates to the use of N-Vinylimidazole and -triazole compounds of the general formula

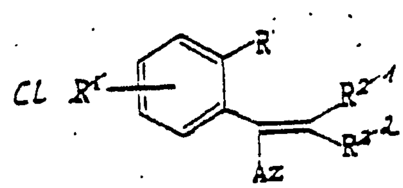

wherein R is a hydroxy, $C_1$-$C_3$ alkoxy, or chloro-benzyloxy group,

$R^1$ and $R^2$ each is a hydrogen atom, a $C_1$-$C_3$ alkyl, $C_1$-$C_3$ alkoxyphenoxy, chlorophenoxy or phenyl group optionally substituted by a methoxy group or one or two chlorine atoms, or $R^1$ and $R^2$ taken together form a 1,5-pentamethylene group.

Az is a 1H-imidazolyl or 1H-1,2,4-triazolyl group, and their acid addition salts for the preparation of a cardiotonic pharmaceutical composition.

The present invention furthermore provides the following novel N-vinyl-imidazole and -triazole compounds:

1-[1-(5-chloro-2-methoxyphenyl)-2-(4-methoxyphenyloxy)vinyl]-1H-imidazole,
1-[1-(5-chloro-2-n-propoxyphenyl)-2-(2,4-dichlorophenyl)vinyl]-1H-imidazole.
1-[1-(5-chloro-2-(4-chlorobenzyloxy)phenyl)-2,2-diethylvinyl]-1H-1,2,4,triazole.
1-[1-(5-chloro-2-hydroxyphenyl)-2,2-diphenylvinyl]-1H-imidazole.
1-[1-(5-chloro-2-methoxyphenyl)-2,2-diphenylvinyl]-1H-imidazole.
1-[1-(5-chloro-2-isopropoxyphenyl)-2,2-diphenylvinyl]-1H-imidazole.
1-[1-(5-chloro-2-methoxyphenyl)-2,2-diphenylvinyl]-1H-1,2,4-triazole.
1-[1-(5-chloro-2-isopropoxyphenyl)-2,2-diphenylvinyl]-1H-1,2,4-triazole.
1-[1-(5-chloro-2-isoproproxyphenyl)cyclohexylidenmethyl]-1H-imidazole.

The compounds of the present invention have an excellent cardiotonic activity. Accordingly the invention also provides a pharmaceutical composition comprising a cardiotonically effective amount of at least one compound of the general formula (I) and one or more pharmaceutically acceptable carriers, diluents and/or excipients.

The process for preparing the compounds of the general formula (I) comprises reacting an acetophenone derivative of the general formula:

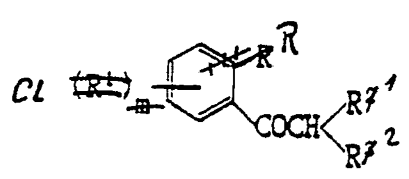

(II)

in which R, $R^1$ and $R^2$ have the same meaning as defined above)
with an azole-introducing agent of the general formula: Az-Q(III)

(wherein Q is -SO-Az, -CO-Az or -SO-Hal, Hal is halogen and Az is as defined above) in a solvent.

This reaction is attained by reacting the acetophenone derivative (II) with the azole-introducing agent such as the thionyl compound, carbonyl componnd or halothionyl compound in solvent at a temperature under cooling (e.g. -10 to 10° C) to room temperature (10 to 30° C) or at an elevated temperature (e.g. to 50 to 120° C). Examples of the solvent are dichloromethane, 1, 2-dichloroethane, dimethyl sulfoxide, acetonitrile, dimethylformamide and chlorbenzene.

Further the compounds (I a) containing a phenolic hydroxy group can be converted into the compounds(I b) having a phenolic ether group in a conventional manner, for example, by reacting ( I a) with an etherifying agent, R-W( IV) such as $C_1$.$C_3$ alkyl halide or a chlorobenzyl halide. The reaction may be performed in the presence of a base such as sodium hydroxide, potassium hydroxide, potassium carbonate, triethylamine or pyridine in a solvent such as methanol, ethanol, dimethylformamide, acetone, toluene, dioxane or dimethylsulfoxide at a temperature from 0° C to 120° C, preferably 30 to 80° C.

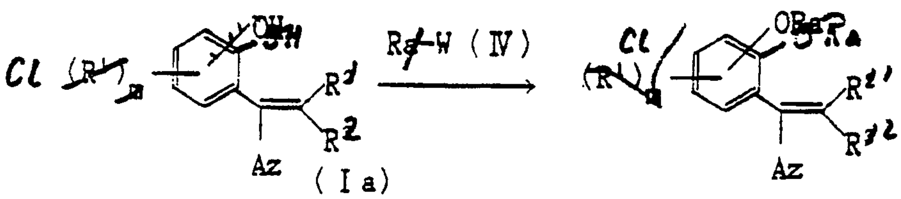


( I a) ( I b)

(wherein R, Az, $R^1$ and $R^2$ is as defined above).

The terms used in the above definition will be illustratively explained below.

The term "halogen" includes fluorine, chlorine, bromine and iodine.

The term "$C_1$-$C_3$ alkyl" herein employed refers to a straight or branched saturated aliphatic hydrocarbon radical such as methyl, ethyl, n-propyl or isopropyl.

The term "$C_1$-$C_3$ alkoxy" represents methoxy, ethoxy, n-propoxy, isopropoxy.

The starting compound (II) can be prepared from the corresponding phenols by Fries Rearrangement or Friedel Crafts Reaction.

The starting compounds (II) can also be prepared by the process shown in Referential Examples 8 to 12.

The compounds (I) can be converted into an acid addition salt and these salts are also included in the scope of the invention.

Illustrative acids which can form such salts are inorganic acids such as hydrohalogenic acid (hydrochloric acid hydrobromic acid, etc.), sulfuric acid, nitric acid, or phosphoric acid, and organic acid such as acetic acid, propionic acid, oxalic acid, malonic acid, succinic acid, glutalic acid, adipic acid, malic acid, maleic acid, fumaric acid, citric acid, benzoic acid, and methanesulfonic acid.

The compounds (I) of the present invention and their acid addition salts can be orally or parenterally administered to humans or animals as cardiotonic drug. For oral dose, tablets, pills, granules, powder capsules, liquid, emulsions, and suspensions are available. For parenteral dose, suppositories and injection preparations are suitable forms.

The preparations may be made by methods well known in the pharmaceutical field, with conventional additives such as vehicles, binders, and lubricants, preparations may be used in the form of solutions in distilled water, physiological saline, or Ringer's solution, or in the form of suspensions in sesame oil.

The following Examples, and Preparations illustrate the invention,

The abbreviations used in the Examples, and Tables represent the following meaning:

Me: methyl; Et: ethyl; n-Pr: n-propyl; ; i-Pr: iso-propyl; Im: 1H-imidazol-1-yl; Tri: 1,2,4-triazol-1-yl; MeOH: methanol; EtOH: ethanol; $CH_2Cl_2$: dichloromethane; $CHCl_3$: chloroform; $Et_2O$: diethyl ether; $AC_2O$: acetone; DMF: dimethylformamide; ; $Et_3N$: triethylamine;; $Na_2SO_4$: anhydrous sodium sulfate; AcOEt: ethyl acetate; (d): decomposition point.

Example 1

1{(5-Chloro-2-hydroxyphenyl)cyclohexylidenemethyl}-1H-imidazole I-1

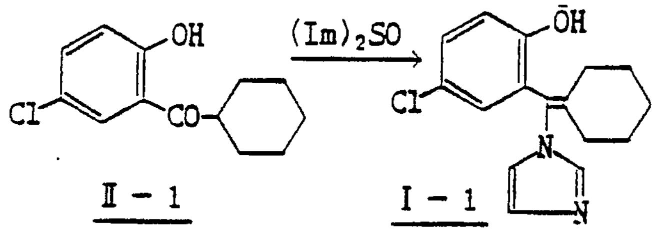


II - 1 I - 1

Into an ice-cooled solution of 3.8 g (55.8 mmol) of imidazole dissolved in 22 ml of dry $CH_2Cl_2$ was dropped 1.65 g (13.9 mmol) of $SOCl_2$. After the mixture was stirred for 5 min. at the same temperature, 2.2 g (9.2 mmol) of 5-chloro-2-hydroxyphenyl cyclohexyl ketone II -1 was added thereto. After stirring for 15 min. at room temperature, the reaction mixture was mixed with ice water and was made alkaline with aqueous solution of $NaHCO_3$ and extracted with $CH_2Cl_2$. The extract was washed with water, and after drying over $Na_2SO_4$, $CH_2Cl_2$ was distilled off. The residue was subjected to silica gel column chromatography being eluted with 3 % MeOH-$CH_2Cl_2$. The eluate was concentrated, and the residue was washed with isopropyl ether and recrystallized from AcOEt to give 1.2 g of the compound I -1 (yield : 45.1%).

m.p. : 217-219 °C

Elemental analysis :

| | |
|---|---|
| Calcd. (%) | ( for $C_{16}H_{17}ClN_2O$ ): |
| | C,66.55; H, 5.93; Cl,12.28; N,9.70. |
| Found (%) | C,66.46; H,6.08; Cl,12.37; N,9.63. |

Examples 2-9

Compounds shown in Table 1 were obtained in the same manner as in Example 1. The compound numbers were assigned correspondingly to the Example numbers as I -2 to I -9.

Table 1. (No. 1)

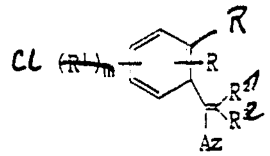

( I )

| Ex. No. | Az | R | Cl | $R^1$ | $R^2$ | m. p. (°C) |
|---|---|---|---|---|---|---|
| 2 | Im | 2-OH | 5-Cl | Et | Et | 170~171 |
| 3 | Tri | 2-OH | 5-Cl | Et | Et | 158~159 |
| 4 | Tri | 2-OH | 5-Cl | 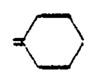 | | 154~155 |
| 5 | Im | 2-OH | 5-Cl | 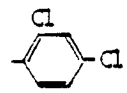 | H | 216~217 |
| 6 | Im | 2-OH | 5-Cl | H | 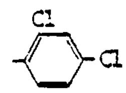 | 193~194 |
| 7 | Im | 2-OH | 5-Cl | Ph | Ph | >270 |
| 8 | Tri | 2-OH | 5-Cl | Ph | Ph | 217~220 |
| 9 | Im | 2-OH | 5-Cl | 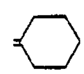 | | 217~219 |

## Example 10

1-[(5-Chloro-2-isopropoxyphenyl)cyclohexylidenmethyl]-1H-imidazole.

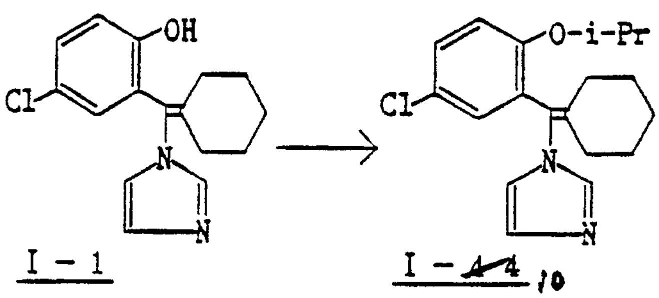

I - 1 I - 10

The compound I-1 (300 mg) obtained in Example 1 was dissolved in 3 ml of dry DMF and added with 75 mg (1.5 mmol) of 50 % NaH (oily suspension) under ice-cooling and stirring. 270 mg (1.5 mmol) of isopropyl iodide was added to the mixture, which was reacted for 30 min. at room temperature. The reaction product was poured into ice water and extracted with AcOEt. The organic layer was washed with water, dried over $Na_2SO_4$, and concentrated in vacuum. The residue was subjected to silica gel-column chloromatography, being eluted with $CH_2Cl_2$-MeOH (100:0 to 97.3 v/v). The fraction containing the objective substance was collected and concentrated. The residue was recrystallized from isopropyl ether-petroleum ether to give the titled compound I-44 .

Yield : 52.4 %

m.p. : 84 - 85 °C; HCl salt, m.p. : 183 - 184 °C
Elemental analysis :

| | | |
|---|---|---|
| Calcd. (%) | (for $C_{19}H_{23}ClN_2O$) : | |
| | C, 68,96; H, 7.01; Cl, 10.72; N, 8.47, | |
| Found (%) : | C, 69.32; H, 7.21; Cl, 10.87; N, 8.42. | |

Examples 11

The compounds shown in Table 2 were obtained in the same manner as in Example 10. The compound numbers were assigned correspondingly to the Example numbers as I -11 to I -29.

Table 2. (No. 1)

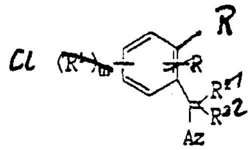

( I )

| Ex. No. | Az | R | ~~(R1)m~~ Cl | R1 | R2 | m.p. (°C) |
|---|---|---|---|---|---|---|
| 11 (~~52~~) | Im | 2-O-i-Pr | 5-Cl | Et | Et | 173~174 (COOH)₂ |
| 12 (~~53~~) | Tri | 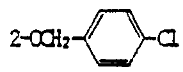 | 5-Cl | Et | Et | 129~130 |
| 13 (~~54~~) | Tri | 2-OMe | 5-Cl | Et | Et | 73~74 |
| 14 (~~55~~) | Tri | 2-O-i-Pr | 5-Cl | Et | Et | 69~70 |
| 15 (~~56~~) | Im | 2-OMe | 5-Cl | 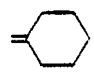 | | 121~122 |
| 16 (~~57~~) | Tri | 2-OMe | 5-Cl | 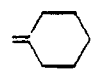 | | 129~130 |
| 17 (~~58~~) | Tri | 2-O-i-Pr | 5-Cl | 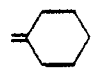 | | 89~90 |
| 18 (~~59~~) | Tri | 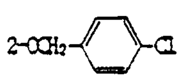 | 5-Cl | 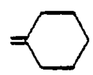 | | 105~107 |
| 19 (~~60~~) | Im | 2-OMe | 5-Cl | Ph | Ph | 120~121 |
| 20 (~~61~~) | Im | 2-O-i-Pr | 5-Cl | Ph | Ph | 118~119 |
| 21 (~~62~~) | Tri | 2-OMe | 5-Cl | Ph | Ph | 142~143 |
| 22 (~~63~~) | Tri | 2-O-i-Pr | 5-Cl | Ph | Ph | 120~121 |
| 23 (~~64~~) | Tri | 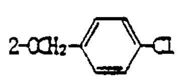 | 5-Cl | Ph | Ph | 133~134 |
| 24 (~~65~~) | Im | 2-O-n-Pr | 5-Cl | H | 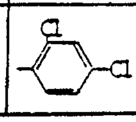 | 123~124 |
| 25 (~~66~~) | Im | 2-O-i-Pr | 5-Cl | 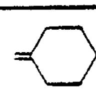 | | 84~85 |
| 26 (~~67~~) | Im | 2-O-n-Pr | 5-Cl | 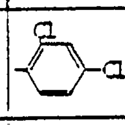 | H | 137.5~138.5 (COOH)₂ |

Table 2. (No. 2)

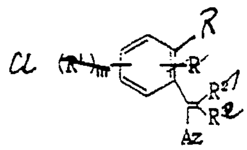

( I )

| | Ex. No. | Az | R | Cl (R¹)n | R¹ | R² | m. p. (°C) |
|---|---|---|---|---|---|---|---|
| 27 | 98-(1) | Im | 2-OMe | 5-Cl | H | 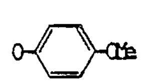 | 油 NMR δ ($CDCl_3$) 3.72 (s, 3H), 3.75 (s, 3H), 6.79~7.73 (m, 10H), 7.7 (s, 1H) |
| 27 | 98-(2) | Im | 2-OMe | 5-Cl | H | 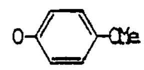 | 175~178 $(COOH)_2$ |
| 28 | 99 | Im | 2-OMe | 5-Cl | H | 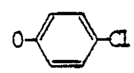 | 102~104 |
| 29 | 100 | Im | 2-OMe | 5-Cl | 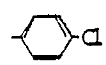 | H | 86~87 |

Preparation:

| | | |
|---|---|---|
| 1-((5-chloro-2-hydroxyphenyl)cyclohexylidenemethyl)-1H-imidazole I -1 | ..... | 100 mg |
| Wheat starch | ..... | 250 mg |
| Magnesium stearate | ..... | 50 mg |

These ingredients were admixed and filled in capsules.

Effect of the Invention:

The compounds (I) of the invention or their acid addition salts show cardiotonic action and are useful as drugs.

The followings are pharmacologically activities of Compound (I) of the invention based on the experiments examples.

Experiment

Positive inotropic effect in the isolated right atrium of guinea-pigs.

Test Method

Male and female guinea-pigs weighing 400-600 mg were used. After a blow on the head, guinea pigs were bled to death by the carotid artery dissection, and their right atria were isolated. The isolated right atria were held in Magnus vessel filled with Krebs-Ringer carbonate solution saturated with 95 % oxygen and 5 % carbon dioxide at 30 °C. Spontaneous contractions of atria were recorded on a polygraph (Nihon Kohden, Ltd.) with an FD pick up ( SB-1T, Nihon Kohden, Ltd.). The number of beats was counted by a pulse counter from the spontaneous contraction.

The compound to be tested was dissolved in methanol or methanesulfonic acid, and its solution was put into the Magnus vessel at a concentration of $10^{-5}$ g/ml.

As a control, amrinone [5-amino-(3,4'-bipyridin)-6(1H)-one] was used at a concentration of $10^{-5}$ g/ml.

| Compd. No. | Changes (%) | |
|---|---|---|
| | Contractile Force | Number of Beats |
| I - ~~98~~ 27 -(1) | + 73.8 | + 14.7 |
| I - ~~65~~ 24 | + 130.3 | + 35.1 |
| I - ~~58~~ 12 | + 77.0 | + 32.2 |
| I - ~~11~~ 8 | + 73.7 | + 25.7 |
| I - ~~60~~ 19 | + 146.1 | + 44.5 |
| I - ~~61~~ 20 | + 144.2 | + 29.3 |
| I - ~~62~~ 21 | + 99.4 | + 45.6 |
| Control | + 77.3 | + 17.1 |

Results

## Claims

1. Use of N-Vinyl-imidazole and -triazole compounds of the general formula

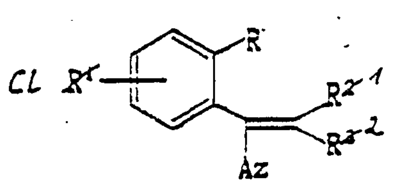

(1)

wherein R is a hydroxy, $C_1$-$C_3$ alkoxy, or chloro-benzyloxy group,

$R^1$ and $R^2$ each is a hydrogen atom, a $C_1$-$C_3$ alkyl, $C_1$-$C_3$ alkoxyphenoxy, chlorophenoxy or phenyl group optionally substituted by a methoxy group or one or two chlorine atoms, or $R^1$ and $R^2$ taken together from a 1,5-pentamethylene group.

Az is a 1H-imidazolyl or 1H-1,2,4-triazolyl, group and their acid addition salts for the preparation of a cardiotonic pharmaceutical composition.

2. A compound according to Claim 1, namely 1-[- 1-(5-chloro-2-methoxyphenyl)-2-(4-methoxyhenyloxy)-vinyl]-1H-imidazole.

3. A compound according to Claim 1, namely 1-[- 1-(5-chloro-2-n-proproxyhenyl)-2-(2,4-dichlorophenyl)-vinyl]-1H-imidazole.

4. A compound according to Claim 1, namely 1-[- 1-(5-chloro-2-(4-cnloroblenzyloxy)phenyl)-2,2-diethylvinyl]-1H-1,2,4,triazole.

5. A compound according to Claim 1, namely 1-[- 1-(5-chloro-2-hydroxyphenyl)-2,2-dienylvinyl]-1H-imidazole.

6. A compound according to Claim 1, namely 1-[- 1-(5-chloro-2-methoxyhenyl)-2,2-diphenylvinyl]-1H-imidazole.

7. A compound according to Claim 1, namely 1-[- 1-(5-chloro-2-isopropoxyphenyl)-2,2-diphenylvinyl]-1H-imidazole.

8. A compound according to Claim 1, namely 1-[- 1-(5-chloro-2-methoxyphenyl)-2,2-diethylvinyl]-1H-1,2,4,triazole.

9. A compound according to Claim 1, namely 1-[- 1-(5-chloro-2-isopropoxyphenyl)-2,2-diethylvinyl]-1H-1,2,4,triazole.

10. A compound according to Claim 1, namely 1-[- 1-(5-chloro-2-isopropoxyphenyl)cyclohexylidenmethyl]-1H-imidazole.

11. A pharmaceutical composition comprising a cardiotonically effective amount of at least one compound according to Claims 2-11 and one or more pharmaceutically acceptable carriers, diluents and/or excipients.

## Revendications

1. Utilisation de composés de N-vinylimidazole et-triazole de formule générale

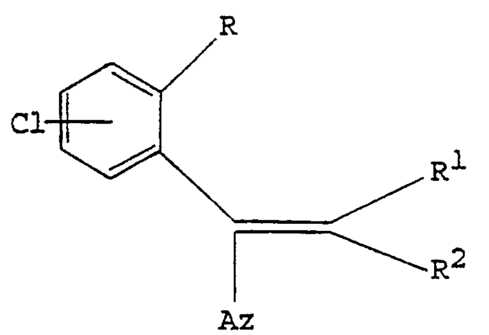

(I)

dans laquelle R est un groupe hydroxy, alcoxy en $C_1$-$C_3$ ou chloro-benzyloxy,
$R^1$ et $R^2$ sont chacun un atome d'hydrogène, un groupe alkyle en $C_1$-$C_3$, alcoxyphénoxy en $C_1$-$C_3$, chlorophénoxy ou phényle éventuellement substitué par un groupe méthoxy ou un ou deux atomes de chlore, ou bien $R^1$ et $R^2$ pris ensemble forment un groupe 1,5-pentaméthylène,
Az est un groupe 1H-imidazolyle ou 1H-1,2,4-triazolyle, et de leurs sels d'addition d'acide pour la préparation d'une composition pharmaceutique cardiotonique.

2. Composé selon la revendication 1, à savoir le 1-[1-(5-chloro-2-méthoxyphényl)-2-(4-méthoxyphényloxy) vinyl]-1H-imidazole.

3. Composé selon la revendication 1, à savoir le 1-[1-(5-chloro-2-n-propoxyphényl)-2-(2,4-dichlorophényl) vinyl]-1H-imidazole.

4. Composé selon la revendication 1, à savoir le 1-[1-(5-chloro-2-(4-chlorobenzyloxy)phényl)-2,2-diéthylvinyl]-1H-1,2,4-triazole.

5. Composé selon la revendication 1, à savoir le 1-[(5-chloro-2-hydroxyphényl)-2,2-diphénylvinyl]-1H-imidazole.

6. Composé selon la revendication 1, à savoir le 1-[1-(5-chloro-2-méthoxyphényl)-2,2-diphénylvinyl]-1H-imidazole.

7. Composé selon la revendication 1, à savoir le 1-[1-(5-chloro-2-isopropoxyphényl)-2,2-diphénylvinyl]-1H-imidazole.

8. Composé selon la revendication 1, à savoir le 1-[1-(5-chloro-2-méthoxyphény1)-2,2-diphénylvinyl] -1H-1,2,4-triazole.

9. Composé selon la revendication 1, à savoir le 1-[1-(5-chloro-2-isopropoxyphényl)-2,2-diphénylvinyl]-1H-1,2,4-triazole.

10. Composé selon la revendication 1, à savoir le 1-[1-(5-chloro-2-isopropoxyphényl)-cryclohexylidèneméthyl]-1H-imidazole.

11. Composition pharmaceutique comprenant une quantité efficace du point de vue cardiotonique d'au moins un composé selon les revendications 2 à 11 et un ou plusieurs véhicules, diluants et/ou excipients acceptables du point de vue pharmaceutique.

## Ansprüche

1. Verwendung von N-Vinylimidazol und -triazolverbindungen der allgemeinen Formel (I)

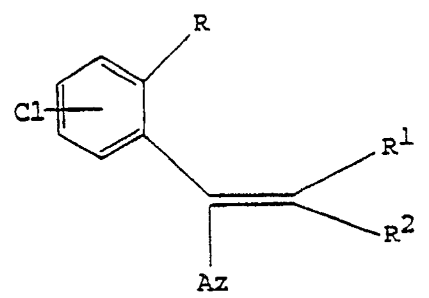

(I)

in der R eine Hydroxygruppe, einen $c_1$-$c_3$-Alkoxyrest oder eine chlorbenzyloxygruppe darstellt,
$R^1$ und $R^2$ jeweils ein Wasserstoffatom, einen $c_1$-$C_3$-Alkyl-, $c_1$-$c_3$-Alkoxyphenoxy-, chlorphenoxy- oder Phenylrest, gegebenenfalls substituiert mit einer Methoxygruppe oder einem oder zwei Chloratomen, darstellen, oder $R^1$ und $R^2$ zusammen eine 1,5-Pentamethylengruppe bilden, und
Az eine 1H-Imidazolyl- oder 1H-1,2,4-Triazolylgruppe darstellt,
sowie ihrer Säureadditionssalze, zur Herstellung eines cardiotonischen Arzneimittels.

2. Verbindung nach Anspruch 1, nämlich 1-[1-(5-Chlor-2-methoxyphenyl) -2-(4-methoxyphenyloxy)-vinyl]-1-1H-imidazol.

3. Verbindung nach Anspruch 1, nämlich 1-[1-(5-Chlor-2-n-propoxyphenyl)-2-(2,4-dichlorphenyl)-vinyl]-1H-imidazol.

4. Verbindung nach Anspruch 1, nämlich 1-[1-(5-Chlor-2-(4-chlorbenzyloxy)-phenyl)-2,2-diethylvinyl]-1H-1,2,4-triazol.

5. Verbindung nach Anspruch 1, nämlich 1-[(5-Chlor-2-hydroxyphenyl)-2,2-diphenylvinyl]-1H-imidszol.

6. Verbindung nach Anspruch 1, nämlich 1-[1-(5-Chlor-2-methoxyphenyl)-2,2-diphenylvinyl]-1H-imidazol.

7. Verbindung nach Anspruch 1, nämlich 1-[1-(5-Chlor-2-isopropoxyphenyl)-2,2-diphenylvinyl]-1H-imidazol.

8. Verbindung nach Anspruch 1, nämlich 1-[1-(5-Chlor-2-methoxyphenyl)-2,2-diphenylvinyl]-1H-1,2,4-triazol.

9. Verbindung nach Anspruch 1, nämlich 1-[1-(5-Chlor-2-isopropoxyphenyl)-2,2-diphenylvinyl]-1H-1,2,4-triazol.

10. Verbindung nach Anspruch 1, nämlich 1-[1-(5-Chlor-2-isopropoxyphenyl)-cyclohexylidenmethyl]-1H-imidazol.

11. Arzneimittel, enthaltend eine cardiotonisch wirksame Menge von mindestens einer Verbindung der Ansprüche 2 bis 11 und einen oder mehrere pharmazeutisch verträgliche Träger, Verdünnungsmittel und/oder Exzipientien.